(19) Europäisches Patentamt
European Patent Office
Office européen des brevets

(11) **EP 2 752 653 A1**

(12) **EUROPEAN PATENT APPLICATION**
published in accordance with Art. 153(4) EPC

(43) Date of publication:
**09.07.2014 Bulletin 2014/28**

(51) Int Cl.:
***G01N 21/64*** (2006.01)

(21) Application number: **12827884.3**

(22) Date of filing: **23.08.2012**

(86) International application number:
**PCT/JP2012/071331**

(87) International publication number:
**WO 2013/031643 (07.03.2013 Gazette 2013/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2011 JP 2011187601**

(71) Applicant: **Olympus Corporation**
**Shibuya-ku**
**Tokyo 151-0072 (JP)**

(72) Inventor: **NISHIKAWA Kazutaka**
**Tokyo 151-0072 (JP)**

(74) Representative: **Schicker, Silvia**
**Wuesthoff & Wuesthoff**
**Patent- und Rechtsanwälte**
**Schweigerstrasse 2**
**81541 München (DE)**

(54) **METHOD FOR DETECTING A TARGET PARTICLE IN BIOSAMPLE CONTAINING PANCREATIC JUICE**

(57)  Provided is a method for detecting a target particle in a biosample containing pancreatic juice, the method enabling the detection in a solution that has a lower concentration or number density of the target particles than the level possible for conventional photoanalysis techniques. This method comprises: a probe-binding step for preparing a sample solution, which contains a biosample containing pancreatic juice and a fluorescent probe capable of binding to a target particle, and binding the fluorescent probe to the target particle in the biosample; and a calculation step for calculating the number of molecules of the target particles bound to the fluorescent probes by the scanning molecule counting method. A light emission property of emitted light is different between a state where the fluorescent probe is bound to the target particle and a state where the fluorescent probe is present alone. In a state where the fluorescent probe is bound to the target particle, the fluorescent probe emits fluorescence having a wavelength of 600 nm or longer.

FIG. 1A

EP 2 752 653 A1

**Description**

TECHNICAL FIELD

The present invention relates to a method for detecting a target particle in a biosample containing pancreatic juice, with use of an optical system which can detect light from a micro region in a solution, such as an optical system of a confocal microscope and a multiphoton microscope.

Priority is claimed on Japanese Patent Application No. 2011-187601, filed August 30, 2011, the content of which is incorporated herein by reference.

BACKGROUND ART

According to the developments in photometric measurement techniques in recent years, detection and measurement of faint light at a single photon or single fluorescent molecule level have become possible by using an optical system of a confocal microscope and a super high sensitive light detection technique capable of photon counting (single photon detection). Thus, various devices or methods of performing the detection of intermolecular interaction or binding/dissociating reaction of biological molecules etc. using such a faint light measurement technique, have been proposed.

For example, in Fluorescence Correlation Spectroscopy (FCS, see e.g. Patent Documents 1 and 2 and Non-patent Documents 1 to 3), the fluorescence intensity from fluorescence molecules or fluorescently labeled molecules (fluorescent molecules etc.), entering and exiting a micro region in a sample solution, is measured by using an optical system of a laser confocal microscope and a photon counting technique. From the autocorrelation function value of the measured fluorescence intensity, the average dwell time (translational diffusion time) of the fluorescent molecules etc. and the average number of the dwelling molecules in the micro region are determined. Based on the average dwell time of the fluorescent molecules etc. and the average number of the dwelling molecules in the micro region, the information on the speed of the movement, the size, and the concentration of the fluorescent molecules etc. can be acquired. Furthermore, various phenomena, such as a change of a molecular structure or size, a binding/dissociating reaction, or dispersion/aggregation of molecules, can be detected. Note that, the above-mentioned micro region means a focal region to which the laser light of the microscope is condensed, and is called a "confocal volume".

Moreover, in Fluorescence Intensity Distribution Analysis (FIDA, e.g. Patent Document 3) or Photon Counting Histogram (PCH, e.g. Patent Document 4), a histogram is produced of the fluorescence intensity of fluorescent molecules etc., entering and exiting a confocal volume, which is measured in the same manner as that of FCS. Then, the average value of the characteristic brightness of the fluorescent molecules etc. and the average number of molecules dwelling in the confocal volume are calculated by fitting a statistical model formula to the distribution of the histogram. Then, based on the information thereof, the change of the molecular structure or size, binding/dissociative conditions, and dispersion/aggregation conditions of molecules can be estimated.

Furthermore, Patent Documents 5 and 6 propose methods of detecting fluorescent substances based on a time progress of a fluorescence signal of a sample solution measured by using an optical system of a confocal microscope. Patent document 7 proposes a signal calculation processing technique for detecting the existences of fluorescent fine particles in a flow or on a substrate by measuring faint light from the fluorescent fine particles flowing through a flow cytometer or fluorescent fine particles fixed on the substrate using a photon counting technique.

Especially, according to the method employing the fluorescence measurement technique of a micro region by using an optical system of a confocal microscope and a photon counting technique, such as FCS and FIDA, the concentration and the amount of the sample required for the measurement may be extremely small (the amount for use in one measurement may be about several tens of $\mu$L at most), as compared with the prior art, and the measuring time is also shortened a lot (in one measurement, a measuring process taking a time of a second order is repeated several times). Thus, these techniques are expected to be a strong tool enabling an experiment or a test at low cost or quickly in comparison with conventional biochemical methods, especially in cases of conducting an analysis of rare or expensive samples which are often used in the field of medical and biological research and development, and in cases of conducting handling a large number of specimens, such as clinical diagnosis of diseases or the screening of bioactive substances.

In medical diagnoses, body fluids are important biosamples to understand the condition of organs. Pancreatic juice, a type of such body fluids, is also an important biosample to understand the condition of the pancreas, which has been used for test and researches such as cytological diagnoses and measurements of various types of biomolecules. For example, it is expected to be possible to diagnose the onset of pancreatic cancer by checking a tumor marker in pancreatic juice.

Usually, a wide variety of substances are contained in a biosample such as pancreatic juice, and autofluorescent substances are also often contained therein. There have been test methods which utilize autofluorescence in biosamples. For example, a diagnostic device has been studied, which utilizes a phenomenon in which a difference occurs in the intensity distribution of autofluorescence emitted from diseased tissue and normal tissue by the irradiation of excitation light, detects autofluorescence emitted from a biosample, and analyzes this autofluorescence to thereby identify changes in the tissue characteristics involved in various kinds of diseases. On the long-wavelength side in the wavelength region of autofluorescence emitted from biological tissue by the irradiation of excitation light, there is a wavelength region which exhibits a largely distinct intensity due to a difference in the tissue characteristics of a biological body. However, the maximum intensity of autofluorescence emitted from biological tissue exists in a wavelength region on the short-wavelength side in a vicinity of 480 nm, and the intensity of autofluorescence in the long-wavelength region is faint. In order to more accurately detect the intensity of such faint autofluorescence in the long-wavelength region, for example, a device described in Patent Document 8 is reported. In this device, first excitation light for generating autofluorescence in biological tissue and second excitation light having a wavelength in a medium waveband of the wavelength region of the autofluorescence to be emitted from the biological tissue by the irradiation of this excitation light are irradiated on the biological tissue at the same time. By so doing, the emission intensity of autofluorescence in the wavelength region on the long-wavelength side is enhanced. As a result, the proportion of noise components mixed in this autofluorescence in the wavelength region on the long-wavelength side can be relatively reduced.

However, in the case where an attempt is made to detect target particles in a biosample by the photoanalysis technique after artificially labeling with a luminescent probe, non-specific signals are generated due to various kinds of impurities, such as autofluorescent substances, derived from the biosample. Hence, for example, in Patent Document 9 is disclosed a method for detecting a target particle in a biosample which includes blood, by labeling the target particles with a chromophor which radiates light of wavelengths from red to near infrared spectrum in a range of 550 to 1300 nm, upon the detection by the photoanalysis technique. By using light in a wavelength region of 550 to 1300 nm for the detection of target particles, the generation of non-specific signals due to endogenous autofluorescent substances such as hemoglobin can be suppressed.

PRIOR ART DOCUMENTS

PATENT DOCUMENTS

Patent document 1: Japanese Unexamined Patent Application, First Publication No. 2005-098876
Patent document 2: Japanese Unexamined Patent Application, First Publication No. 2008-292371
Patent document 3: Japanese Patent No. 4023523
Patent document 4: PCT International Publication No. WO 2008-080417
Patent document 5: Japanese Unexamined Patent Application, First Publication No. 2007-20565
Patent document 6: Japanese Unexamined Patent Application, First Publication No. 2008-116440
Patent document 7: Japanese Unexamined Patent Application, First Publication No. H04-337446
Patent document 8: Japanese Unexamined Patent Application, First Publication No. 2001-198079
Patent document 9: Specification of U.S. Patent Application Publication No. 2005/0171434

NON-PATENT DOCUMENTS

Non-patent document 1: Masataka Kinjo; "Protein, Nucleic acid, and Enzyme" Vol. 44, No. 9, pages 1431-1438, 1999.
Non-patent document 2: Meyer-Alms; "Fluorescence Correlation Spectroscopy" edt. R. Rigler, Springer, Berlin, pages 204-224, 2000.
Non-patent document 3: Noriko Kato, et al. "Gene & Medicine", Vol. 6, No. 2, pages 271-277, 2002.

DISCLOSURE OF INVENTION

PROBLEMS TO BE SOLVED BY THE INVENTION

In the above-mentioned photoanalysis techniques, such as FCS, FIDA, and PCH, generally speaking, the magnitude of timewise fluctuation of the measured fluorescence intensity is computed by a statistical process, and various characteristics of fluorescent molecules etc., entering and exiting a micro region in a sample solution, are determined based on the magnitude of the fluctuation. Thus, in order to obtain a significant result in the above-mentioned photoanalysis technique, it is preferable to prepare the concentration or number density of fluorescent molecules etc. serving as the object of observation in a sample solution so that fluorescent molecules etc. of a number enabling a statistical process will enter and exit a micro region during a one-time measurement period of a length of a second order in an equilibrium. Suitably, it is preferable to prepare the concentration or number density so that about one fluorescent molecule etc. will always exist in the micro region. Typically, it is preferable that the concentration of fluorescent molecules etc. is about 1 nM or more, since the volume of a confocal volume is about 1 fL.

In other words, when the concentration or number density of target particles in a sample solution is much lower than the level enabling a statistical process, for example, much lower than 1 nM, a condition may occur in which the object of observation rarely enters the micro region within the period of the measurement. In this case, the measurement result of the fluorescence intensity would include a condition in which no object of observation exists at all in the micro region over a long period of time, and also the observable amount of significant fluorescence intensity would decrease. As a result, any significant or precise analysis result can not be obtained in the photoanalysis technique based on the statistical fluctuation of the fluorescence intensity as described above.

In the method of detecting fluorescent substances by using an optical system of a confocal microscope described in Patent Documents 5 and 6, it is possible to determine the presence or absence of a fluorescent molecule etc. serving as the object of observation in a sample, from the presence or absence of generation of a fluorescence signal having a significant intensity in the period of measurement over several seconds, without performing a statistical process of the fluorescence intensity fluctuation as described above. As a result, it is possible to obtain a correlation between the frequency of fluorescence signals having a significant intensity and the number of fluorescent molecules etc. in a sample. In particular, in Patent Document 6, it is suggested that the generation of a random flow agitating the inside of the sample solution improves the detection sensitivity.

However, these methods are not different from conventional methods in the point that it is only possible to detect the existences of fluorescent molecules etc. entering a micro region at random by diffusion or a random flow. Therefore, it is not possible to grasp the behavior of a particle such as a fluorescent molecule etc. in the micro region. Neither, for instance, the counting of particles nor the quantitative computing of the concentration or number density of particles has been possible.

The technique described in Patent Document 7 is to detect individual existences of fluorescent fine particles in a flow in a flow cytometer or fluorescent fine particles fixed on a substrate. This technique described in Patent Document 7 is not a technique to detect particles, such as molecules or colloids, being dissolved or dispersed in a normal condition in a sample solution, in other words, particles moving at random in a sample solution. Accordingly, with the technique described in Patent Document 7, it is not possible to quantitatively compute out the concentration or number density of particles dissolved or dispersed in a sample solution.

Moreover, the technique of Patent Document 7 includes processes, such as the measurement in a flow cytometer or the treatment of fixing fluorescence particles on a substrate. Thus, the amount of the sample necessary for the test should be much larger than those of the cases of photoanalysis techniques, such as FCS, FIDA, and PCH, and complicated and advanced operational techniques should be requested for a person who conducts the test.

It is an object of the present invention to provide a method for detecting a target particle in a biosample containing pancreatic juice, without needing a statistical process as executed in photoanalysis techniques, such as FCS, FIDA, and PCH. In this method, it is possible with the novel photoanalysis technique to detect the condition or characteristic of target particles even in a case where the concentration or number density of the target particles is lower than the level that can be treated by these photoanalysis techniques such as FCS, FIDA, and PCH.

MEANS TO SOLVE THE PROBLEMS

The inventor of the present invention and others have conducted earnest studies to solve the above-mentioned problems, which resulted in the following findings. When indirectly detecting a particle dispersed and moving at random in a sample solution with, as an indicator, light emitted from fluorescent probes bound to the particle, it is possible by detecting the particles bound to the fluorescent probes with use of the scanning molecule counting method, to sensitively detect the particles bound to the fluorescent probes even if the concentration of the target particles in the sample solution is extremely low. When detecting a target particle in a biosample which contains pancreatic juice, it is possible by having the wavelength of fluorescence for detecting the fluorescent probe bound to the target particle to be 600 mn or longer, to detect the target particles while effectively suppressing the generation of non-specific signals even if autofluorescent substances derived from pancreatic juice is contained in the sample solution. The inventor of the present invention has discovered the above-mentioned findings, and completed the present invention.

Here, the scanning molecule counting method is a novel photoanalysis technique proposed in Japanese Patent Application No. 2010-044714.

Hereunder shows the aspects of the present invention.

(1) A method for detecting a target particle in a biosample containing pancreatic juice, wherein the method comprises:
(a) a probe-binding step for preparing a sample solution which contains a biosample containing pancreatic juice and a fluorescent probe capable of binding to the target particle, and binding the fluorescent probe to the target particle contained in the biosample, in the sample solution; and (b) a calculation step for calculating the number of molecules of the target particles bound to the fluorescent probes existing in the sample solution prepared in the step (a); a light emission property of light emitted from the fluorescent probe is different between a state where the fluorescent probe is bound to the target particle and a state where the fluorescent probe is present alone; and the wavelength of emitted light in a state where the fluorescent probe is bound to the target particle is 600 nm or longer; and the calculating of the number of molecules of the target particles bound to the fluorescent probes in the step (b) is carried out by: a moving step for, with use of an optical system of a confocal microscope or a multiphoton microscope, moving a position of a light detection region of the optical system in the sample solution; a detection step for, while moving the position of the light detection region of the optical system in the sample solution, detecting a light signal emitted from the fluorescent probes in the state of being bound to the target particles in the light detection region, thereby individually detecting the target particles bound to the fluorescent probes; and a counting step for counting the number of the individually detected target particles bound to the fluorescent probes to thereby count the number of molecules of the target particles detected during the moving of the position of the light detection region.

(2) The method for detecting a target particle according to (1), wherein the position of the light detection region is moved at a predetermined speed, in the moving step for moving the position of the light detection region.

(3) The method for detecting a target particle according to either one of (1) and (2), wherein the position of the light detection region is moved at a speed higher than the speed of diffusional movement of the target particles bound to the fluorescent probe, in the moving step for moving the position of the light detection region.

(4) The method for detecting a target particle according to any one of (1) to (3), wherein it is detected that one target particle bound to the fluorescent probe has entered the light detection region, based on the shape of the chronologically detected light signal, in the detection step for detecting the light signals from the respective target particles bound to the fluorescent probes from the detected light, and individually detecting the target particles bound to the fluorescent probes.

(5) The method for detecting a target particle according to any one of (1) to (4), wherein the wavelength of fluorescence emitted from the fluorescent probe in the state of being bound to the target particle is from 630 nm to 1300 nm.

(6) The method for detecting a target particle according to any one of (1) to (5), wherein: the fluorescent probe has an energy donor site and an energy acceptor site, which produce a fluorescence energy transfer phenomenon when these sites are close to each other, and the distance between the energy donor site and the energy acceptor site is different between a state where the fluorescent probe is bound to the target particle and a state where the fluorescent probe is not bound to the target particle; and a light emission property of light emitted from the fluorescent probe is different between a state where the fluorescent probe is bound to the target particle and a state where the fluorescent probe is present alone.

(7) The method for detecting a target particle according to any one of (1) to (6), wherein: the target particle is a nucleic acid; and the fluorescent probe is a single stranded nucleic acid molecule which is specifically hybridizable with the target particle, and which is bound with at least either one of a fluorescent substance serving as an energy donor and a substance serving as an energy acceptor in a fluorescence energy transfer phenomenon.

EFFECT OF THE INVENTION

In the scanning molecule counting method employed in the method for detecting a target particle of the aspects of the present invention (hereunder, referred to as the method for detecting a target particle of the present invention), no statistical process such as computing the fluorescence intensity fluctuation is executed. Therefore, with the method for detecting a target particle of the present invention, the target particles to be analyzed in a sample can be detected even if the target particles exist at an extremely small amount in the sample. Furthermore, in the method for detecting a target particle of the present invention, the influence of autofluorescent substances contained as impurities can be eliminated, by detecting the fluorescent probe bound to the target particle as a light signal of fluorescence having a wavelength of 600 nm or longer in the scanning molecule counting method. Therefore, as a result of these, the target particles in a biosample containing pancreatic juice can be highly precisely detected.

BRIEF DESCRIPTION OF THE DRAWINGS

FIG. 1A is a schematic diagram of the internal structure of a photoanalysis device for the scanning molecule counting method.
FIG. 1B is a schematic diagram of a confocal volume (an observation region of a confocal microscope).
FIG. 1C is a schematic diagram of a mechanism for changing the direction of a mirror 7 to move the position of a light detection region in a sample solution.

FIG. 2A is a schematic diagram explaining the principle of light detection by the photoanalysis technique for the scanning molecule counting method.

FIG. 2B is a schematic diagram of the chronological change of the measured light intensity.

FIG. 3A is a drawing of a model in the case that target particles are passing across a light detection region owing to Brownian motion.

FIG. 3B is a diagram showing an example of the chronological change of the photon counts (light intensity) in the situation shown in FIG. 3A.

FIG. 4A is a drawing of a model in the case that target particles are passing across a light detection region by moving the position of the light detection region in a sample solution at a speed higher than the speed of diffusional movement of the target particles.

FIG. 4B is a diagram showing an example of the chronological change of the photon counts (light intensity) in the situation shown in FIG. 4A.

FIG. 5 is a flow chart showing the procedure of processes for counting the particles from the chronological change of the photon counts (light intensity) measured by the scanning molecule counting method.

FIG. 6A is a diagram explaining an example of the signal processing step of the detected signals in the procedure of processes for counting the particles from the chronological change of the photon counts (light intensity) measured by the scanning molecule counting method.

FIG. 6B are diagrams explaining an example of the signal processing step of the detected signals in the procedure of processes for counting the particles from the chronological change of the photon counts (light intensity) measured by the scanning molecule counting method.

FIG. 7 shows an example of the actually measured photon count data by the scanning molecule counting method (bar graph), a curve obtained by smoothing the data (dotted line), and Gauss functions fitted on the peak existence regions (solid line). In the diagram, the signals appended as "noise" are disregarded as signals derived from a noise or a contaminant.

BEST MODE FOR CARRYING OUT THE INVENTION

Firstly, the scanning molecule counting method is described. In the scanning molecule counting method, while scanning the inside of a sample solution with a micro region, light emitted from a luminescent particle dispersed and moving at random in the sample solution (hereunder, referred to as "luminescent particle") in the micro region, is detected when the luminescent particle passes across the inside of the micro region. Thus, the scanning molecule counting method makes possible, by individually detecting luminescent particles in the sample solution one by one, the counting of the luminescent particles, and the acquisition of the information about the concentration or number density of the luminescent particles in the sample solution. In the scanning molecule counting method, similarly to the photoanalysis techniques such as FIDA, the amount of the sample required for the measurement may be extremely small (e.g., several tens of μ L). Moreover, the measuring time is short in the scanning molecule counting method. Furthermore, in the scanning molecule counting method, it is possible to quantitatively detect the characteristics, such as its concentration or number density, of the luminescent particles having lower concentration or number density than the cases of the photoanalysis techniques such as FIDA.

The term "a particle dispersed and moving at random in a sample solution" means an atom or a molecule or an aggregate thereof dispersed or dissolved in the sample solution, or such a particle (which may or may not emit light), as well as being a particle moving freely in the solution owing to the Brownian motion without being fixed to a substrate or the like.

The luminescent particle means a particle which emits light by fluorescence, phosphorescence, chemiluminescence, bioluminescence, light scattering, etc. In the method for detecting a target particle of this embodiment, a combined substance of a target particle and a fluorescent probe is the luminescent particle.

In this embodiment, the term "light detection region" of an optical system of a confocal microscope or a multiphoton microscope, is a micro region where light is detected in the confocal microscope or the multiphoton microscope. It corresponds to the region to which illumination light is condensed when the illumination light is given from an object lens. This light detection region is determined especially in accordance with the spatial relationship of an object lens and a pinhole in a confocal microscope.

In the scanning molecule counting method, the detection of light is chronologically performed while moving the position of the light detection region in the sample solution, that is, while scanning the inside of the sample solution with the light detection region. Then, when the moving light detection region includes a luminescent probe bound to or associated with a randomly moving particle, light from the luminescent probe is detected. By so doing, the existence of one particle is detected (the luminescent probe may be dissociated from a particle to be detected (target particle) at the time of the light detection after once being bound to the particle, depending on the embodiment of the experiment). Then, the light signal from each luminescent probe is individually detected with chronologically detected light. By so doing, the existence of a particle (bound to the luminescent probe) is individually and chronologically detected one by one, and various information on the condition of the particle within the solution are acquired. Concretely, for example, the above-mentioned structure may be such that the number of the particles detected during the moving of the position of the light detection region is counted by counting the number of the individually detected particles (the counting of particles). According to this structure, by combining the number of the particles and the moving amount of the position of the light detection region, the information on the number density or concentration of the particles in the sample solution can be acquired. Especially, if the whole volume of the moving track of the position of the light detection region is determined by an arbitrary way, for example, such as moving the position of the light detection region at a predetermined speed, the number density or concentration of the particles can be concretely computed. Of course, instead of determining directly the absolute number density value or concentration value, the relative ratio of the number density or concentration, to a plurality of sample solutions or a standard sample solution that becomes a reference of a concentration or a number density, may be computed. Moreover, the scanning molecule counting method is designed to move the position of the light detection region by changing the optical path of the optical system. Because of this, the light detection region moves quickly, and substantially no mechanical vibration nor hydrodynamic action will be generated in the sample solution. For this reason, it is possible in the scanning molecule counting method to conduct light measurement under a stable condition where the particles as the object of detection are not influenced by such a mechanical action (when a vibration or flow is applied to the inside of the sample solution, the physical characteristics of the particles might be changed.). Moreover, since there is no need of making the sample solution circulate in the scanning molecule counting method, the measurement and the analysis are possible with an extremely small amount (about one to several tens of μL) of the sample solution, similarly to FCS, FIDA, and the like.

In the above-mentioned step of individually detecting the particle, the judgment from the chronologically detected light signal regarding whether or not a luminescent probe bound to one particle has entered the light detection region may be done based upon the shape of the chronologically detected light signal. In the embodiments, typically, the structure may be designed to detect that a luminescent probe bound to one particle has entered the light detection region when a light signal having an intensity greater than a predetermined threshold value is detected. Note that, in this embodiment, the luminescent probe bound to one particle includes a state where one luminescent probe is bound to one particle, a state where a plurality of luminescent probes are bound to one particle, and a state where luminescent probe(s) is/are dissociated from one particle after being bound to one particle, depending on the embodiment.

Moreover, in the above-mentioned step of moving the position of the light detection region, the speed of moving the position of the light detection region in the sample solution may be appropriately modified based on the characteristic of the luminescent probe bound to the particle, or the number density or concentration in the sample solution. As understood by ones ordinarily skilled in the art, the manner of light detected from the luminescent probe bound to the particle may be different depending on the characteristic, or the number density or concentration in the sample solution. Especially, when the speed of moving the light detection region is higher, the amount of light obtained from the luminescent probe bound to one particle will be reduced. Therefore, it is preferable to appropriately modify the speed of moving the light detection region so that the light from the luminescent probe bound to one particle can be measured with good precision or good sensitivity.

Furthermore, in the above-mentioned step of moving the position of the light detection region, the speed of moving the position of the light detection region in the sample solution is preferably set to be higher than the speed of diffusional movement (the average moving speed of particles owing to the Brownian motion) of the luminescent probes bound to the particles serving as the object of detection (that is, luminescent probes in a state of being bound to the target particles in the method for detecting a target particle of the present invention). As explained above, in the scanning molecule counting method, light emitted from a luminescent probe is detected when the light detection region passes through the position where the luminescent probe bound to one particle exists, thereby detecting the luminescent probe individually. However, if the luminescent probe bound to the particle moves at random owing to the Brownian motion in the solution to thereby enter and exit the light detection region multiple times, the light signal (showing the existence of the particle desired to be detected) will be detected from one luminescent probe multiple times. This makes it difficult to establish a correspondence between the detected light signal and the existence of one particle desired to be detected. Therefore, as described above, the speed of moving the light detection region is set higher than the speed of diffusional movement of the luminescent probes bound to the particles, by which it becomes possible to establish a correspondence between a luminescent probe bound to one particle and one light signal (showing the existence of the particle). Since the speed of diffusional movement is different depending upon the luminescent probes bound to particles, it is preferable to appropriately modify the speed of moving the light detection region according to the characteristics (especially, the diffusion constant) of the luminescent probes bound to particles. In this embodiment, concretely, the speed of moving the light detection region is set higher than the speed of diffusional movement of the luminescent probes in a state of being bound to the target particles.

The optical path of the optical system for moving the position of the light detection region may be changed in an arbitrary way.

For example, the optical path may be changed by using a galvanomirror employed in the laser scan type light microscope so that the position of the light detection region can be changed. The movement track of the position of the light detection region may be set arbitrarily, for example, it may be selectable from circular, elliptical, rectangular, straight, and curvilinear ones.

The scanning molecule counting method is designed so that its light detecting mechanism itself has a structure to detect light from a light detection region in a confocal microscope or a multiphoton microscope similarly to the cases of photoanalysis techniques such as FIDA. Thus, the amount of a sample solution may be extremely small in the same way. However, since no statistical process such as computing the fluorescence intensity fluctuation is executed in the scanning molecule counting method, the photoanalysis technique of the scanning molecule counting method is applicable to a sample solution in which the number density or concentration of particles is much lower than the level required for the photoanalysis techniques such as FIDA.

Moreover, in the scanning molecule counting method, each of the particles dispersed or dissolved in a solution is individually detected. Thus, it is possible, by using the information, to quantitatively count the particles, calculate the concentration or number density of the particles in the sample solution, or acquire the information about the concentration or number density. That is, according to the scanning molecule counting method, since the particles are detected one by one by establishing a one-on-one correspondence between a particle passing through the light detection region and a detected light signal, the counting of particles dispersed and moving at random in a solution becomes possible. Therefore, according to the scanning molecule counting method, it becomes possible to determine the concentration or number density of particles in a sample solution more precisely as compared with the conventional art. Actually, according to the method for detecting a target particle of this embodiment to determine the particle concentration by individually detecting fluorescent probes in a state of being bound to the target particles and counting the number thereof, the detection of the target particles is possible even if the concentration of the fluorescent probes bound to the target particles in the sample solution is much lower than the concentration which can be determined based upon the fluorescence intensity measured by a fluorescence spectrophotometer or a plate reader.

Furthermore, according to the manner of scanning the inside of a sample solution with a light detection region by changing the optical path of an optical system, the inside of the sample solution is observed uniformly or under a condition where the sample solution is mechanically stable without applying a mechanical vibration nor a hydrodynamic action to the sample solution. By so doing, for example, the reliability of the quantitative detection result is improved as compared with a case where a flow is generated in a sample (when a flow is given to a sample, it is difficult to give an always uniform flow speed and the device structure becomes complicated. Moreover, the required amount of the sample largely increases, and the particle, the luminescent probe, the combined body thereof, or another substance, in the solution may be deteriorated or denaturalized by the hydrodynamic action owing to the flow.). Moreover, the measurement can be carried out under a condition where there are no influences or artifacts due to dynamic action against the particles serving as the object of detection in the sample solution.

<The structure of a photoanalysis device for the scanning molecule counting method>

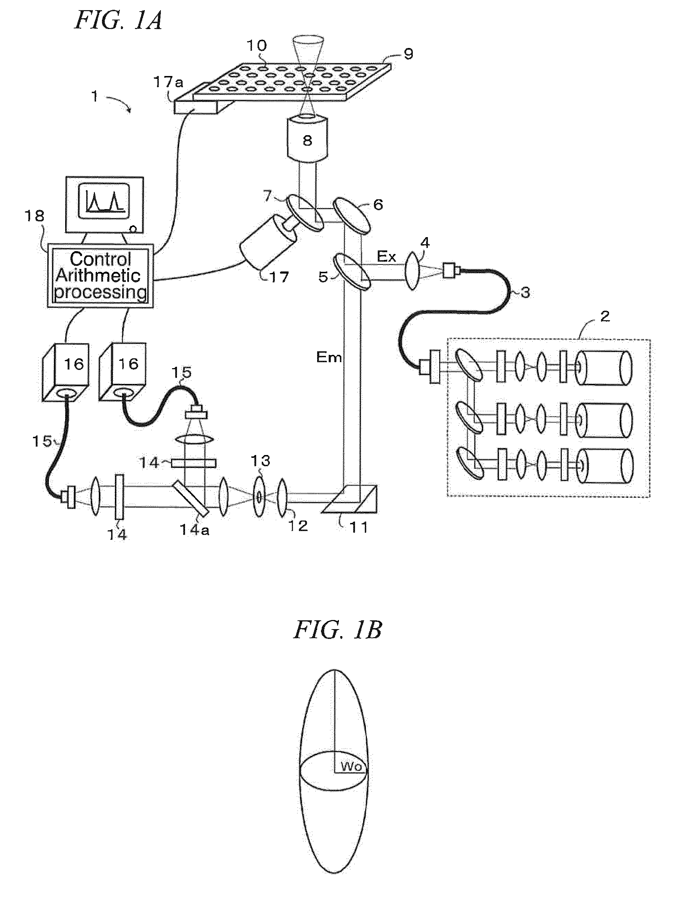

In the basic structure, the scanning molecule counting method can be realized with a photoanalysis device formed by combining an optical system of a confocal microscope and a photodetector as schematically illustrated in FIG. 1A, with which FCS, FIDA, etc. can be performed. Referring to FIG. 1A, the photoanalysis device 1 consists of an optical system 2-17 and a computer 18 for acquiring and analyzing data as well as controlling the operation of each part in the optical system. The optical system of the photoanalysis device 1 may be the same as the optical system of a usual confocal microscope. In the optical system, laser light emitted from a light source 2 and transmitted through the inside of a single mode fiber 3 (Ex) forms light diverging to be radiated at an angle decided by an inherent numerical aperture (NA) at the emitting end of the fiber. The radiated light forms a parallel beam with a collimator 4, is reflected on a dichroic mirror 5 and reflective mirrors 6 and 7, and enters an object lens 8. Typically, above the object lens 8 is placed a sample container or a micro plate 9 having wells 10 arranged therein, to which one to several tens of $\mu$L of a sample solution is dispensed. The laser light emitted from the object lens 8 is focused in the sample solution in the sample container or the well 10, forming a region having a strong light intensity (excitation region). In the sample solution, particles serving as the object of observation and luminescent probes to be bound to the particles, typically, molecules to which a light emitting label such as a fluorescent dye is attached, are dispersed or dissolved. When a particle which has been bound to or associated with a luminescent probe (it may be a luminescent probe dissociated from a particle after once being bound to the particle, depending on the embodiment) enters the excitation region, the luminescent probe is excited and emits light during the period. The emitted light (Em) passes through the object lens 8 and the dichroic mirror 5, is reflected on the mirror 11, and condensed by a condenser lens 12. The condensed light passes through the pinhole 13, transmits through a barrier filter 14 (where light components only in a specific wavelength band region are selected), and is introduced into a multimode fiber 15, reaching to a photodetector 16. Then, after the conversion into chronological electric signals, the signals are inputted into the computer 18, where the processes for optical analyses are executed in manners explained later. As known to ones skilled in the art, in the above-mentioned structure, the pinhole 13 is located at a conjugate position of the focal position of the object lens 8. Thereby, as schematically shown in FIG. 1B, only the light emitted from the focal region of the laser light, i.e., the inside of the excitation region, passes through the pinhole 13 while the light from regions other than the excitation region is blocked. The focal region of the laser light illustrated in FIG. 1B is a light detection region in this photoanalysis device, whose effective volume is usually about 1-10 fL (Typically, the light intensity is spread in accordance with a Gaussian type or Lorentz type distribution having the peak at the center of the region. The effective volume is a volume of an approximate ellipsoid bordering a surface where the light intensity is reduced to $1/e^2$ of the peak intensity.), which is called "confocal volume". Moreover, light from one combined body of the particle and the luminescent probe, or the luminescent probe, for example, faint light from one or several fluorescent dye molecule(s), is detected in the scanning molecule counting method. For this reason, preferably, a super high sensitive photodetector, usable for the photon counting, is used for the photodetector 16. Moreover, on the stage (not shown) of the microscope may be provided a stage position changing apparatus 17a for moving the horizontal position of the micro plate 9, in order to change the well 10 to be observed. The operation of the stage position changing apparatus 17a may be controlled by the computer 18. According to this structure, quick measurement can be achieved even if a plurality of specimens are present.

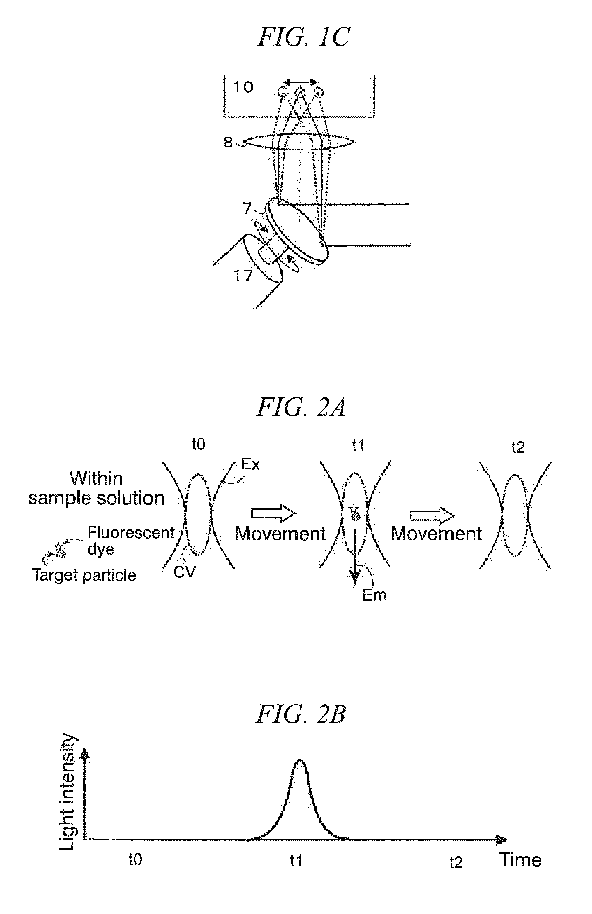

Furthermore, in the optical system of the above-mentioned photoanalysis device, the optical path of the optical system is changed to scan the inside of the sample solution with the light detection region. In other words, there is provided a mechanism for moving the position of the focal region (i.e., the light detection region) within the sample solution. Regarding this mechanism for moving the position of the light detection region, for example, as schematically illustrated in FIG. 1C, a mirror deflector 17 for changing the direction of the reflective mirror 7 may be employed. This mirror deflector 17 may be the same as that of a galvanomirror device equipped on a usual laser scan type microscope. Moreover, in order to attain a desired moving pattern of the position of the light detection region, the mirror deflector 17 is driven in harmony with the light detection of the photodetector 16 under the control of the computer 18. The movement track of the position of the light detection region may be arbitrarily selected from circular, elliptical, rectangular, straight, and curvilinear ones, or a combination of these (The program in the computer 18 may be designed so that various moving patterns can be selected.). Although not illustrated, the position of the light detection region may be moved in the vertical direction by moving the object lens 8 up and down. As noted, by changing the optical path of the optical system to move the position of the light detection region instead of moving the sample solution, substantially no mechanical vibration or hydrodynamic action will be generated in the sample solution. Consequently it becomes possible to eliminate the influence of a dynamic action on the object of observation, which achieves a stable measurement.

In the case that a combined body of a particle and a luminescent probe, or a luminescent probe, emits light owing to multiple photon absorption, the above-mentioned optical system is used as a multiphoton microscope. In that case, since the light is emitted only from the focal region of the excitation light (light detection region), the pinhole 13 may be removed. Moreover, in the case that a combined body of a particle and a luminescent probe, or a luminescent probe, emits light owing to a chemiluminescence or bioluminescence phenomenon without excitation light, the optical system 2-5 for generating excitation light may be omitted. In the case that a combined body of a particle and a luminescent probe, or a luminescent probe, emits light owing to phosphorescence or scattered light, the above-mentioned optical system of the confocal microscope is used as it is. Furthermore, in the photoanalysis device 1, as shown in FIG. 1A, a plurality of excitation light sources 2 may be provided so that the wavelength of the excitation light can be selected appropriately in accordance with the wavelength of light for exciting a combined body of a particle and a luminescent probe, or a luminescent probe. Similarly, a plurality of photodetectors 16 may also be provided so that, in a case that the sample contains a plurality of kinds of combined bodies of particles and luminescent probes, or luminescent probes, whose wavelengths differ from each other, the respective lights from them can be detected separately in accordance with the wavelengths.

<The principle of the photoanalysis technique of the scanning molecule counting method>

Spectral analysis techniques, such as FIDA, are advantageous from the point that the required amount of the sample is extremely small and a test can be executed promptly as compared with the conventional biochemical analytical techniques. However, in the spectral analysis techniques such as FIDA, the concentration and characteristics of the target particles are principally calculated based on the fluorescence intensity fluctuation. In order to obtain highly precise measurement results, the concentration or number density of the target particles in a sample solution should be at a level where about one target particle always exists in the light detection region CV during the fluorescence intensity measurement so that a significant light intensity (photon count) can be always detected in the period of the measurement. If the concentration or number density of the target particles is lower than that, for example, at the level where the target particle rarely enters the light detection region CV, a significant light intensity (photon count) would appear only in a part of the period of the measurement, and thus, highly precise calculation of the light intensity fluctuation would become difficult. Moreover, if the concentration of the target particles is much lower than the level where about one target particle always exists inside the light detection region during the measurement, the calculation of the light intensity fluctuation would be easily influenced by the background, and the period of the measurement has to be long in order to obtain a significant quantity of the light intensity data, which is sufficient for the calculation. On the other hand, in the scanning molecule counting method, it is possible to detect the characteristics of the target particles, such as its number density or concentration, even if the concentration of the target particles is lower than the level required in the spectral analysis techniques such as FIDA.

In the photoanalysis technique of the scanning molecule counting method, briefly speaking, as a process to be executed, light detection is performed while moving the position of the light detection region CV in a sample solution, that is, while scanning the inside of the sample solution with the light detection region CV, by driving the mechanism for moving the position of the light detection region (mirror deflector 17) to change the optical path as schematically drawn in FIG. 2A.

By so doing, for example, as shown in FIG. 2A, during the moving of the light detection region CV (in the drawing, time t0-t2), when the light detection region CV passes through a region where one particle (in the drawing, a fluorescent dye as a luminescent probe is bound thereto) exists (t1), a significant light intensity (Em) is detected as drawn in FIG. 2B. In this way, the moving of the position of the light detection region CV and the light detection as described above are executed, and each significant light intensity appearing as illustrated in FIG. 2B during the period is detecting one by one. Because of this, the particle bound to the luminescent probe is detected individually. By counting the number of the detected particles, the information about the number, or concentration or number density, of the particles existing in the measured region can be acquired. In the principle of this photoanalysis technique of the scanning molecule counting method, no statistical calculation process, such as the calculation of the fluorescence intensity fluctuation, is conducted and the particles are detected one by one. It should be understood that, because of this, it is possible to acquire the information about the concentration or number density of particles even with a sample solution whose concentration of the particles to be observed is too low to perform a sufficiently precise analysis in FIDA or the like.

Moreover, according to the method of individually detecting particles in a sample solution and counting them, like the scanning molecule counting method, it is possible to measure a lower concentration than the case of measuring the concentration of fluorescently labeled particles from the fluorescence intensity measured with a fluorescence spectrophotometer or a plate reader. In the case of measuring the concentration of certain fluorescently labeled particles with a fluorescence spectrophotometer or a plate reader, usually, an assumption is made such that the fluorescence intensity is proportional to the concentration of the fluorescently labeled particles. However, in that case, when the concentration of the fluorescently labeled particles is significantly low, the amount of noise signals relative to the amount of signals of the light emitted from the fluorescently labeled particles (deterioration of the S/N ratio) becomes large, and the proportionality relation between the concentration of the fluorescently labeled particles and the amount of light signals collapses. As a result, the precision of the determined concentration value deteriorates. On the other hand, in the scanning molecule counting method, noise signals are eliminated from the detected result in the step of detecting the signals corresponding to the respective particles from the detected light signals, and the concentration is calculated by counting only the signals corresponding to the respective particles. Therefore, it is possible to detect a lower concentration than the case of detecting the concentration under the assumption that the fluorescence intensity is proportional to the concentration of the fluorescently labeled particles.

Furthermore, in a case that a plurality of luminescent probes are bound to one target particle, then according to the method of individually detecting particles in a sample solution and counting them, like in the scanning molecule counting method, the precision of the measurement of a particle concentration on a higher side of the particle concentration is also improved compared to the conventional method of determining the concentration under the assumption that the fluorescence intensity is proportional to the concentration of the fluorescently labeled particles. In a case that a plurality of luminescent probes are bound to one target particle, and when a certain amount of luminescent probes are added to the sample solution, the number of the luminescent probes bound to one particle relatively decreases as the concentration of the target particles increases. In this case, because the fluorescence intensity per one target particle is decreased, the proportionality relation between the concentration of the fluorescently labeled particles and the amount of light collapses, which deteriorates the precision of the determined concentration value. On the other hand, in the scanning molecule counting method, the influence of the reduction in the fluorescence intensity per one particle is small in the step of detecting the signals corresponding to the respective particles from the detected light signals, and the concentration is calculated from the number of the particles. Thus, it is possible to detect a higher concentration than the case of detecting the concentration under the assumption that the fluorescence intensity is proportional to the concentration of the fluorescently labeled particles.

<Measurement of the light intensity of a sample solution by the scanning molecule counting method>

The measurement of the light intensity in the photometric analysis of the scanning molecule counting method may be executed in the same manner as that of the measurement process of the light intensity in FCS or FIDA, except for driving the mirror deflector 17 to move the position of the light detection region within the sample solution (scanning inside of the sample solution) during the measurement. In the operation process, typically, after dispensing a sample solution into the well(s) 10 of the micro plate 9 and placing it on the stage of the microscope, a user inputs a command of starting a measurement into the computer 18. Subsequently, the computer 18 illuminates the light detection region in the sample solution with excitation light and starts to measure the light intensity, according to the programs (a procedure for changing the optical path in order to move the position of the light detection region in the sample solution, and a procedure for detecting light from the light detection region during the moving of the position of the light detection region) stored in a storage device (not shown). During this measurement, under the control of the operation process of the computer 18 according to the programs, the mirror deflector 17 drives the mirror 7 (galvanomirror) to move the position of the light detection region in the well 10. Simultaneously with this, the photodetector 16 sequentially converts the detected light into an electric signal and transmits it to the computer 18. The computer 18 generates the chronological light intensity data from the transmitted light signals and stores it in an arbitrary manner. The photodetector 16 is typically a super high sensitive photodetector which can detect an arrival of a single photon. Thus, the detection of light is the photon counting executed in the manner of measuring sequentially the number of photons which arrive at the photodetector for every predetermined unit time (BINTIME), for example, every 10 micro seconds, during a predetermined period of time. The chronological light intensity data may be chronological photon count data.

The speed of moving the position of the light detection region during the measurement of the light intensity may be a predetermined speed which is arbitrarily set, for example, experimentally or in order to meet the purpose of the analysis. In a case of acquiring the information on the number density or concentration based on the number of detected target particles, the size or volume of the region through which the light detection region has passed is required. Therefore, the moving of the position of the light detection region is executed in a manner enabling the grasping of the moving distance. Note that, the interpretation of the measurement result will be easy if the elapsed time during the measurement is proportional to the moving distance of the position of the light detection region. Thus, basically, it is preferable that the moving speed is constant, although the speed is not to be limited thereto.

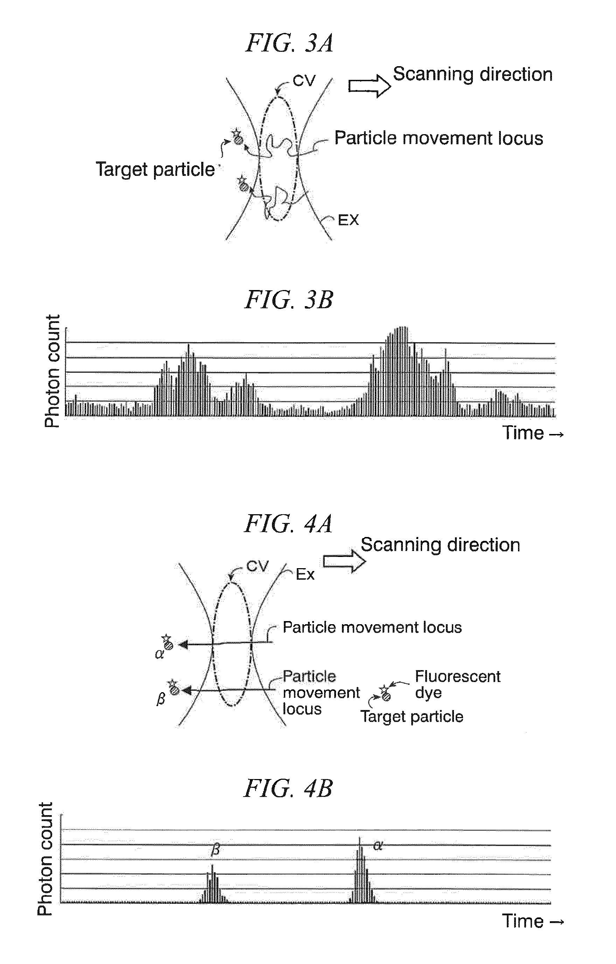

By the way, in order to quantitatively and precisely execute the individual detection of the target particles from the measured chronological light intensity data or the counting of the number of the target particles, it is preferable to set the speed of moving the position of the light detection region, to a value higher than the moving speed in the random motion, i.e., the moving speed owing to the Brownian motion of the target particle (More strictly, a combined body of a particle and a luminescent probe, or a luminescent probe having been freed from a particle by decomposition after being bound thereto. In this embodiment, a target particle bound to a fluorescent probe.). Since the target particle in the photoanalysis technique of the scanning molecule counting method is a particle dispersed or dissolved in a solution and moving freely at random, its position moves with time owing to the Brownian motion. Thus, when the speed of moving the position of the light detection region is slower than the movement of a particle owing to the Brownian motion, the particle moves at random in the region as schematically drawn in FIG. 3A. By so doing, the light intensity changes at random as shown in FIG. 3B (as already noted, the excitation light intensity in the light detection region is reduced from the peak at the center of the region toward its outside), which makes it difficult to specify a significant light intensity change corresponding to each target particle.

Therefore, preferably, as drawn in FIG. 4A, the speed of moving the position of the light detection region is set to be higher than the average moving speed of the particles owing to the Brownian motion (speed of diffusional movement) so that each particle passes across the light detection region in an approximately straight line. Thereby, the profile of the change of the light intensity corresponding to each particle becomes almost uniform in the chronological light intensity data as illustrated in FIG. 4B, and the correspondence between each target particle and the light intensity can be easily specified. When a particle passes through the light detection region in an approximately straight line, the profile of the change of the light intensity is similar to the excitation light intensity distribution.

Concretely, the time $\Delta t$ required for a target particle (more strictly, a combined body of a particle and a luminescent probe, or a luminescent probe having been freed from a particle by decomposition after being bound thereto), having a diffusion coefficient D to pass through the light detection region of radius Wo (confocal volume) by the Brownian motion is given from the expression of the relation of mean-square displacement:

$$(2\mathrm{Wo})^2 = 6D \cdot \Delta t \qquad (1)$$

as:

$$\Delta t = (2\mathrm{Wo})^2 / 6D \qquad (2).$$

Thus, the speed of the target particle moving by the Brownian motion (speed of diffusional movement) Vdif, becomes approximately

$$\mathrm{Vdif} = 2\mathrm{Wo}/\Delta t = 3D/\mathrm{Wo} \qquad (3).$$

Therefore, with reference to such Vdif, the speed of moving the position of the light detection region may be set to a sufficiently higher value than Vdif. For example, when the diffusion coefficient of the target particle is expected to be about $D = 2.0 \times 10^{-10}$ m$^2$/s, Vdif will be $1.0 \times 10^{-3}$ m/s, supposing Wo is about 0.62 $\mu$m. Therefore, the speed of moving the position of the light detection region may be set to about 10 times thereof at 15 mm/s. When the diffusion coefficient of the target particle is unknown, an appropriate speed of moving the position of the light detection region may be determined by repeating preliminary experiments with setting various moving speeds of the position of the light detection region in order to find the condition that the profile of the change of the light intensity becomes an expected profile (typically, similar to the excitation light intensity distribution).

<Analysis of light intensity by the scanning molecule counting method>

When the chronological light intensity data of a sample solution are obtained by the above-mentioned processes, an analysis of the light intensity as described below may be executed in the computer 18 through the processes in accordance with the programs stored in a storage device (procedure for detecting light signals individually from respective luminescent particles emitted from detected light).

(i) Detection of one target particle

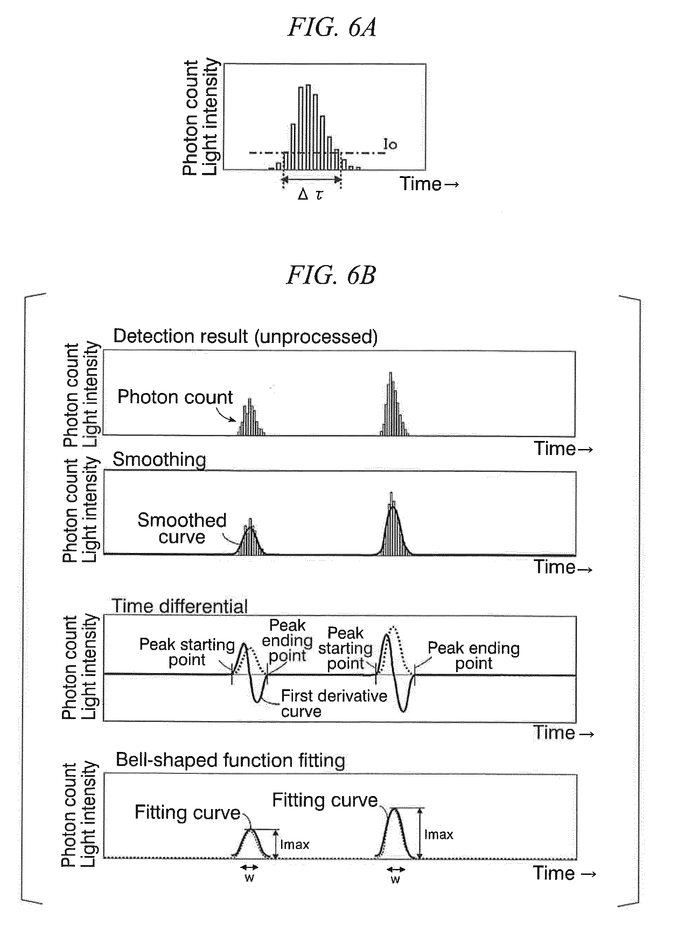

When the track of one target particle in its passing through the light detection region is approximately straight as shown in FIG. 4A, the change of the light intensity corresponding to the particle in the chronological light intensity data has a profile reflecting the light intensity distribution in the light detection region (determined by the optical system) (usually, an approximately bell shape) as schematically drawn in FIG. 6A. Then, in one of the methods for the detection of a target particle, the setting may be such that a threshold value Io is set for the light intensity, and when the time width $\Delta\tau$ for which the light intensity exceeding the threshold value continues is in a predetermined range, the profile of the light intensity is judged to correspond to one particle having passed through the light detection region, and thereby the detection of one target particle will be done. The threshold value Io for the light intensity and the predetermined range for the time width $\Delta\tau$ are determined based on a profile expected as the intensity of the light emitted from a combined body of a target particle and a luminescent probe (or a luminescent probe having been freed from a particle by decomposition after being bound thereto) moving relatively to the light detection region at a predetermined speed. Their concrete values may be set arbitrarily or experimentally, and also may be selectively determined depending upon the characteristics of a combined body of a target particle and a luminescent probe (or a luminescent probe having been freed from a particle by decomposition after being bound thereto).

Moreover, in another method of detection of the target particle, the setting may be such that, when the light intensity distribution in the light detection region can be assumed as a Gaussian distribution:

$$I = A \cdot \exp(-2t^2/a^2) \qquad (4)$$

and when the intensity A and the width a, calculated by fitting the expression (4) to the profile of a significant light intensity (a profile which can be clearly judged not to be a background), are within the respective predetermined ranges, the profile of the light intensity is judged to correspond to one target particle having passed through the light detection region, and thereby the detection of one target particle will be done. When the intensity A and the width a are out of the predetermined ranges, the profile may be disregarded as a noise or a contaminant in the analysis.

(ii) The counting of target particles

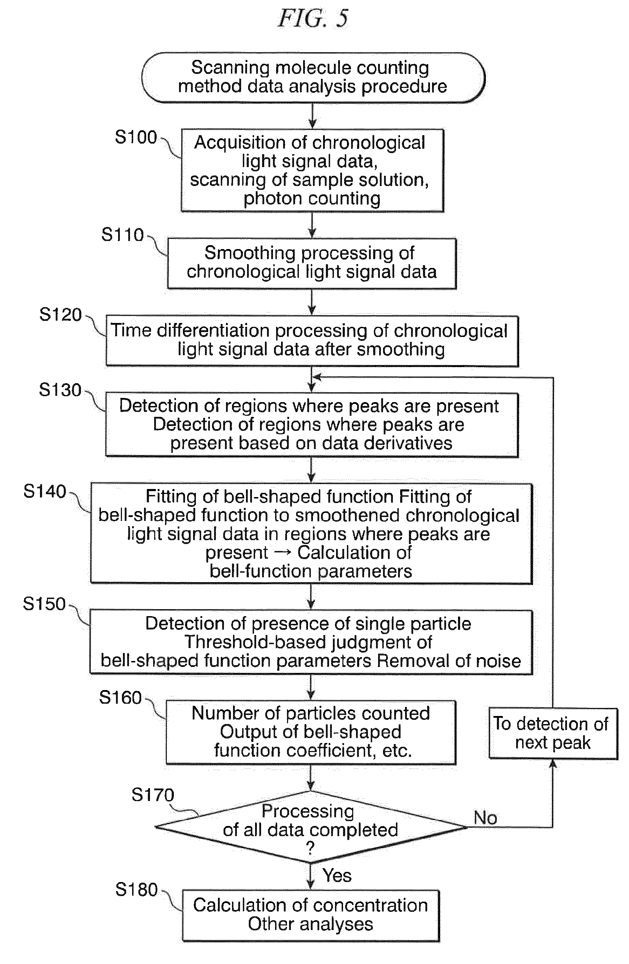

The counting of target particles may be done by counting in an arbitrary way the number of the particles detected by the above-mentioned method of detection of the target particle. However, for a large number of particles, for example, it may be accomplished by the processes illustrated in FIG. 5 and FIG. 6B.

Referring to FIG. 5 and FIG. 6B, one example of ways of performing the counting of particles from the chronological light intensity (photon counts) data is the measurement of the light intensity as explained above. That is, the scanning in the sample solution with the light detection region and the photon counting are performed to acquire chronological light signal data (photon count data) (Step 100). A smoothing process is performed (Step 110, mid-upper row "smoothing" in FIG. 6B) on the chronological light signal data (the upper row "detection result (unprocessed)" in FIG. 6B). Since the light emitted by a combined body of a particle and a luminescent probe, or a luminescent probe, is stochastic, gaps may be generated in data values in minute times. Therefore, such gaps in the data values can be disregarded by the smoothing process. The smoothing process may be done, for example, by the moving average method. In this regard, parameters in executing the smoothing process, e.g., the number of datum points in one time of the averaging, the number of times of moving average, etc. in the moving average method, may be suitably set in accordance with the moving speed (scanning speed) of the position of the light detection region and/or BIN TIME at the time of the light signal data acquisition.

Next, in the chronological light signal data after the smoothing process, in order to detect a time domain in which a significant signal exists (peak existence region), the primary differentiation value with time of the chronological light signal data after the smoothing process is computed (Step 120). As illustrated in the mid-low row "time differential" in FIG. 6B, the change of the value increases during the time when the signal value changes in the time differential value of chronological light signal data. Thereby, the start point and the end point of a significant signal (peak signal) can be determined advantageously by referring to the time differential value.

After that, a significant signal (peak signal) is detected sequentially on the chronological light signal data, and it is judged whether or not the detected peak signal is a signal corresponding to a target particle.

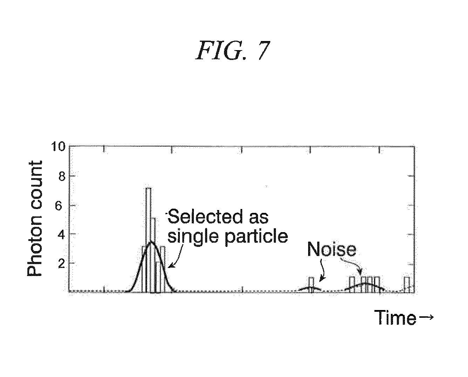

Concretely, first, on the chronological time-differential value data of the chronological light signal data, the starting point and the end point of one peak signal are searched and determined by referring to the time differential value sequentially, by which a peak existence region is specified (Step 130). When one peak existence region has been specified, the fitting of a bell-shaped function is applied to the smoothed chronological light signal data in the peak existence region (the lower row "bell-shaped function fitting" in FIG. 6B). By so doing, parameters in the bell-shaped function, such as the peak intensity, $I_{max}$; the peak width (full width at half maximum), w; the correlation coefficient in the fitting (of the least square method), etc. are calculated (Step 140). In this regard, although the bell-shaped function to be used in the fitting is typically a Gauss function, it may be a Lorentz type function. Then, it is judged whether or not the calculated parameters of the bell-shaped function are within the respective ranges assumed for the parameters of the bell-shaped profile drawn by a light signal detected when one combined body of the particle and the luminescent probe, or one luminescent probe, passes through a light detection region, i.e., whether or not the peak intensity, the peak width, and the correlation coefficient are respectively within the predetermined ranges (Step 150). Thus, the signal, whose calculated parameters of the bell-shaped function are judged to be within the ranges assumed in a light signal corresponding to one combined body of the particle and the luminescent probe, or one luminescent probe, as shown in FIG. 7 left, is judged as a signal corresponding to one target particle. Thereby, it is concluded that one target particle has been detected, and it is counted as one particle (the number of particles is incremented by one: Step 160). On the other hand, a peak signal, whose computed parameters of the bell-shaped function are not within the assumed ranges, as shown in FIG. 7 right, is disregarded as noise.

The search and the judgment of a peak signal in the processes of the above-mentioned steps 130 to 160 are repetitively executed in the whole region of the chronological light signal data, and whenever one target particle is detected, it is counted as one particle. And, when the search of the peak signal in the whole region of the chronological light signal data is completed (Step 170), the count value of particles obtained till then is considered as the number of target particles detected in the chronological light signal data.

(iii) Determination of the number density or concentration of target particles

When the counting of target particles has been done, the number density or concentration of the target particles is determined by using the whole volume of the region which the light detection region has passed through during the acquisition of the chronological light signal data. However, the effective volume of the light detection region varies depending on the wavelength of excitation light or detected light, the numerical aperture of lenses, and the adjustment condition of the optical system, and therefore, it is generally difficult to compute the effective volume of the light detection region from the design parameter values. Accordingly, it is not easy to compute the whole volume of the region which the light detection region has passed through, either. Then, typically, the light intensity measurement, the detection of particles, and the counting thereof may be performed as described above with a solution having a known concentration of the particles (reference solution) under the same condition as that for the measurement of a sample solution to be tested, so that, from the detected number of the particles and the concentration of the particles in the reference solution, the whole volume of the region which the light detection region has passed through, i.e., the relation between the detected number and the concentration of the target particles, can be determined.

Preferably, the particle of the reference solution may be a light emitting label (fluorescent dye etc.) having the same light emission properties as those of a combined body of a particle and a luminescent probe, forming the target particle (or a luminescent probe freed from the target particle after being bound thereto). Concretely, for example, supposing the detected number of the particles is N in a reference solution having a particle concentration C, the whole volume Vt of the region which the light detection region has passed through is given by the following equation.

$$Vt = N/C \qquad (5)$$

Alternatively, a plurality of solutions having different concentrations may be prepared as the reference solutions and the measurement may be executed for each of the solutions, so that the average value of the computed Vt can be employed as the whole volume Vt of the region which the light detection region has passed through. Then, when Vt is given, the number density c of the particles of the sample solution, whose counting result of the particles is n, is given by the following equation.

$$c = n/Vt \qquad (6)$$

In this regard, the volume of the light detection region and the whole volume of the region which the light detection region has passed through may be given by an arbitrary method, for instance, using FCS and FIDA, instead of the above-mentioned method. Moreover, in the photoanalysis device of this embodiment, the information on the relations (expression (5)) between the concentrations C and the numbers N of various standard particles for assumed moving patterns of the light detection region may be previously stored in a storage device of the computer 18, so that a user of the device can appropriately use the stored information on the relation in conducting photometric analysis.

<Method for detecting a target particle>

The method for detecting a target particle of this embodiment is a method for detecting a target particle in a biosample containing pancreatic juice, wherein the target particle labeled with fluorescent probe through binding is detected by the scanning molecule counting method.

Since the scanning molecule counting method is a measurement method capable of measuring luminescent particles by each particle in a condition where the molecules are spaced and apart from each other, the measurement is possible for luminescent particles whose concentration is relatively low in a pM order or lower. For this reason, with the method for detecting a target particle of this embodiment, even if the concentration of target particles as the object of analysis in a sample solution is extremely low, the target particle bound to the fluorescent probe can be counted with good sensitivity.

Furthermore, in the method for detecting a target particle of this embodiment, the wavelength of the light signal to be detected from the fluorescent probe bound to the target particle is 600 nm or longer in the measurement by the scanning molecule counting method. Sample solutions often contain autofluorescent substances originated from biosamples. Particularly, biosamples containing pancreatic juice contain autofluorescent substances having fluorescence wavelengths smaller than 600 nm. In this embodiment, by detecting light signals of 600 nm or longer from among fluorescences emitted from the fluorescent probes bound to the target particles, the generation of non-specific signals due to the autofluorescent substances derived from the biosample containing pancreatic juice is suppressed, by which the fluorescent probe bound to the target particles can be detected with good sensitivity.

Specifically, the method for detecting a target particle of this embodiment has the following steps (a) and (b):

(a) a probe-binding step for preparing a sample solution which contains a biosample containing pancreatic juice and a fluorescent probe capable of binding to the target particle, and binding the fluorescent probe to the target particle contained in the biosample, in the sample solution; and
(b) a calculation step for calculating the number of molecules of the target particles bound to the fluorescent probes existing in the sample solution prepared in the step (a).

Hereunder is a description of the respective steps.

First, as the step (a), a sample solution which contains a biosample containing pancreatic juice and a fluorescent probe capable of binding to the target particle, is prepared, and the target particle contained in the biosample and the fluorescent probe are bound to each other in this sample solution.

Pancreatic juice is a body fluid discharged from the pancreatic duct. In the present invention and the description of this application, the term "biosample containing pancreatic juice" means a sample containing a body fluid which contains a pancreatic juice-derived component. The body fluid which contains a pancreatic juice-derived component can be exemplified by pancreatic juice collected from the pancreas directly through a catheter, a fluid collected from the duodenum (duodenal juice), and the like. Duodenal juice also contains bile discharged from a papillary portion, a fluid originally existing in the duodenum, blood, and the like, in addition to the pancreatic juice. In this regard, the pancreatic juice and the duodenal juice can be collected by a usual method. The biosample containing a pancreatic juice component may be simply composed of such a body fluid which contains a pancreatic juice-derived component, may be a liquid having such a body fluid diluted with an appropriate buffer or the like, or may also be such a body fluid or a diluted liquid thereof with the addition of variety of additives. As the additives, a surfactant or a protease inhibitor for suppressing the decomposition of the pancreatic juice-derived component, etc., a pH adjuster, a pH indicator, or the like can be enumerated.

The target particle is a molecule dispersed and moving at random in a sample solution, and is a molecule as an object to be detected in the sample solution. The target particle may be, for instance, a biological molecule, i.e. a protein, a peptide, a nucleic acid, a nucleic acid analog, a lipid, a sugar chain, an amino acid, etc. or an aggregate thereof, a biological object in a particulate form, i.e. a virus, a cell, etc. or a non-biological particle (i.e., an atom, a molecule, a micelle, a metallic colloid, etc.). The nucleic acid may be DNA, RNA, or an artificially amplified one such as cDNA. The nucleic acid analog can be exemplified by a substance in which a side chain etc. of a natural nucleotide (a nucleotide which exists in nature) such as DNA or RNA is modified with a functional group such as an amino group etc., a substance labeled with a protein, a low molecular compound, etc., and the like. More concretely, the examples include a bridged nucleic acid (BNA), a nucleotide in which an oxygen atom at the 4' position of a natural nucleotide is substituted with a sulfur atom, a nucleotide in which a hydroxyl group at the 2' position of a natural ribonucleotide is substituted with a methoxy group, a hexitol nucleic acid (HNA), a peptide nucleic acid (PNA), and the like.

Moreover, the fluorescent probe for use in the present invention is a substance which can be specifically or non-specifically bound or adsorbed to the target particle, as well as being a substance whose light emission property of emitted light is different between a state where the fluorescent probe is bound to the target particle and a state where the fluorescent probe is present alone, and emits fluorescence having a wavelength of 600 nm or longer in the state where the fluorescent probe is bound to the target particle. The fluorescent probe used in the present invention is excited by light having a wavelength of 600 nm or longer in a state where the fluorescent probe is bound to the target particle, although the fluorescent probe may be a probe which is not excited by light having a wavelength of 600 nm or longer in a state where the fluorescent probe is present alone, or the fluorescent probe may also be a probe which is excited by light having a wavelength of 600 nm or longer both in the state where the fluorescent probe is bound to the target particle and the state where the fluorescent probe is present alone. As the fluorescent probe for use in the present invention, preferred is a probe which emits fluorescence having a wavelength of 630 mn to 1300 nm, and more preferred is a probe which emits fluorescence having a wavelength of 630 mn to 820 nm, in the state where the fluorescent probe is bound to the target particle.

The phrase "the light emission property of a fluorescent probe is different between a state where the fluorescent probe is bound to the target particle and a state where the fluorescent probe is present alone" means that the intensity of light of a specific wavelength is different between the state where the fluorescent probe is bound to the target particle and the state where the fluorescent probe is present alone. By making a difference in the intensity of light of a specific wavelength between the state where the fluorescent probe is present alone and the state where the fluorescent probe is bound to the target particle (for example, making a difference in the fluorescence intensity), both states can be detected distinctively in the scanning molecule counting method.

The fluorescent probe can be produced by, for example, binding a fluorescence substance to a substance which can be specifically or non-specifically bound or adsorbed to the target particle (labeling probe). The fluorescent substance may be used by appropriately selecting from fluorescent dyes for use in FCS, FIDA, etc., quantum dot, and the like.

For example, when the target particle is a nucleic acid or a nucleic acid analog, the fluorescent probe can be exemplified by a substance having a fluorescent substance bound to an oligonucleotide which is hybridizable with the target particle, a nucleic acid-binding protein to which a fluorescence substance is bound, a fluorescent dye molecule which can be bound to a nucleic acid, and the like. This oligonucleotide may be DNA, RNA, or an artificially amplified one such as cDNA, a part or all of which may include a nucleic acid analog which is capable of forming a nucleotide chain or base pairs, similarly to natural nucleic acid bases.

Moreover, when the target particle is a protein, then an antigen or an antibody for the target particle or a ligand or a receptor for the target particle, labeled with a fluorescent substance, can be used as the fluorescent probe. The binding of a fluorescence substance to the substance which can be specifically or non-specifically bound or adsorbed to the target particle such as a nucleic acid or a protein, can be performed by a usual method.

The fluorescent probe for use in this embodiment may be a substance which can be non-specifically bound etc. to the target particle, although a substance which can be specifically bound etc. thereto is preferred in terms of the precision of the detection/quantification of the target particles. The fluorescent probe which can be specifically bound to the target particle may be a substance which can be preferentially bound to the target particle rather than to another substance whose physical or chemical characteristics are similar to those of the target particle, and it is not necessary to be a substance which would not be bound to any other substance than the target particle at all. For example, when the target particle is a nucleic acid, an oligonucleotide labeled with a fluorescence substance for use as a fluorescence probe may have a base sequence which is completely complementary to the base sequence of the target particle, or may have a base sequence which has mismatch(es) against the base sequence of the target particle.

When the target particle is a protein, a dye whose fluorescence intensity or fluorescence wavelength is changed by a change of the surrounding environment due to the binding to the protein (for example, a fluorescent dye such as a hydrophobic probe ANS, MANS, TNS), can be used as the luminescent probe. Moreover, the fluorescent probe may not need to emit light by itself. For example, when the target particle is a nucleic acid or a nucleic acid analog, then an oligonucleotide which is hybridizable with the target particle is used as the fluorescent probe, and a fluorescent double stranded nucleic acid-binding substance which can be specifically bound to a double stranded chain structure may be added together with this fluorescent probe in a sample solution. By so doing, a difference in the light emission property can be made between the state where the fluorescent probe is present alone and the state where the fluorescent probe is bound to the target particle. The fluorescent double stranded nucleic acid-binding substance which can be specifically bound to a double stranded chain structure, can be exemplified by a fluorescent intercalator, a group binder bound with a fluorescent substance, and the like.

Besides, for example, a substance composed of at least two components as well as being a substance which emits fluorescence by a change in the mutual positions of these at least two components due to the binding to the target particle, may be employed as the fluorescent probe. Such a substance can be exemplified by a fluorescent protein which emits strong fluorescence by a change in the structure when being bound to a certain particle, and a molecule (ligand of a complex) which forms a fluorescent metal complex by assembling when being bound to a certain particle. According to this structure, in any case, the fluorescent probe as a single body or the fluorescent probe not bound to the target particle emits almost no light, or even if it emits light, it has a different wavelength from that of the combined body of the target particle and the fluorescent probe. For this reason, it becomes possible to selectively detect light from the combined body of the target particle and the fluorescent probe.

In addition, by utilizing the fluorescence energy transfer phenomenon (FRET), a difference can be made in the light emission property between the fluorescent probe in an unbound state in a sample solution and the fluorescent probe in a state of being bound to the target particle. For example, it is possible to use, as the fluorescent probe, a substance made by binding a fluorescent substance serving as an energy donor and a substance serving as an energy acceptor in FRET (a fluorescent substance and a quencher substance) to a substance which can be bound to the target particle, so that FRET occurs in the state where the fluorescent probe is present alone and FRET does not occur in the state where the fluorescent probe is bound to the target particle. FRET does not occur from the fluorescent probe bound to the target particle, and therefore fluorescence is emitted from the fluorescent substance serving as an energy donor. On the other hand, fluorescence to be emitted from the fluorescent substance serving as an energy donor is not detected or attenuated from the fluorescent probe in an unbound state. Therefore, by detecting the fluorescence emitted from the fluorescent substance serving as an energy donor, the target particle bound to the fluorescent probe can be detected distinctively from the fluorescent probe in an unbound state.

For example, when the target particle is a nucleic acid or a nucleic acid analog, it is possible to preferably use, as the fluorescent probe, a molecular beacon probe made by binding a fluorescent substance serving as an energy donor and a substance serving as an energy acceptor in FRET, to an oligonucleotide which forms an intramolecular structure in a state of a single stranded nucleic acid molecule, so that FRET occurs in a state of a single stranded nucleic acid molecule and FRET does not occur in a state of an associated body formed by hybridization with a different single stranded nucleic acid molecule. In this embodiment, preferred is a substance in which either a fluorescent substance serving as an energy donor or a substance serving as an energy acceptor is bound to the 3'-end side and the other is bound to the 5'-end side, as well as having mutually complementary base sequences in regions on the sides of the 3'-end and the 5'-end, and which forms an intramolecular structure by forming base pairs between these base sequences (a so-called stem loop structure). The mutually complementary regions which form the intramolecular base pairs of the molecular beacon probe may exist so as to interpose the region which is hybridizable with the target particle, and the regions on the sides of the 3'-end and the 5'-end may or may not be regions respectively including the 3'-end or the 5'-end. Moreover, the numbers of bases and the base sequences of the regions forming the base pairs may be at degrees where the stability of the formed base pairs is lower than the stability of the associated body with the target particle, and where the base pairs can be formed under the measurement condition.

Moreover, using a fluorescent double stranded nucleic acid-binding substance which can be specifically bound to a double stranded structure, it is also possible, by causing FRET between this fluorescent double stranded nucleic acid-binding substance and the fluorescent probe-labeled fluorescent substance, to distinguish the fluorescent probe in an unbound state and the fluorescent probe bound to the target particle. That is, either one of the fluorescent double stranded nucleic acid-binding substance or the fluorescent substance for labeling the luminescent probe serves as an energy donor of FRET, and the other one serves as an energy acceptor of this FRET. From the fluorescent probe in an unbound state, fluorescence to be emitted from the fluorescent substance for labeling this luminescent probe is detected. On the other hand, since the fluorescent double stranded nucleic acid-binding substance is bound to the fluorescent probe being bound to the target particle, fluorescence to be emitted from FRET is detected from the combined body. As a result, the combined body can be detected distinctively from the fluorescent probe in an unbound state.

If the amount of the fluorescent intercalator to be inserted between the base pairs of the associated body of the fluorescent probe and the target particle is too large, the background for detecting the fluorescence emitted from FRET becomes so high that the detection precision might be influenced. For this reason, it is preferable to design the fluorescent probe so that the region forming the double strand in the associated body of the fluorescent probe and the target particle is 400 bp or shorter.

Besides, in this embodiment, two types of fluorescent probes may be used. For example, two types of fluorescent probes are designed so that, when the target particle is a nucleic acid or a nucleic acid analog, they can be hybridized with the target particle in mutually adjacent positions, and one fluorescent probe is labeled with a fluorescent substance serving as an energy donor in FRET, and the other fluorescent probe is labeled with a substance serving as an energy acceptor in this FRET. In this case, FRET does not occur in the fluorescent probe in an unbound state. However, when they are bound to the target particle, these two types of fluorescent probes become mutually adjacent and thus FRET occurs. For this reason, the target particle bound to the fluorescent probe can be detected by detecting fluorescence to be emitted from this FRET.

Concretely, in the step (a), firstly, a sample **solution** which contains a biosample containing pancreatic juice and a fluorescent probe capable of binding to the target particle, is prepared. The solvent used therefor is not particularly limited as along as it is a solvent which does not interfere with the detection of light emitted from the fluorescent probe bound to the target particle, and the detection of the fluorescent probe bound to the target particle by the scanning molecule counting method, and the solvent can be used by appropriately selecting from buffers for usual use in this technical field. This buffer can be exemplified by phosphate buffers such as PBS (phosphate buffered saline, pH 7.4), Tris buffer, and the like.

If the target particle and the fluorescent probe can be bound to each other only by having them coexist in the same solution, the target particle and the fluorescent probe can be bound to each other in this sample solution only by incubating this sample solution for a predetermined period of time as required after the preparation of the sample solution.

On the other hand, if the target particle and the fluorescent probe are a nucleic acid or a nucleic acid analog having a double stranded structure, it is preferable to make them associate with each other after denaturing the nucleic acid etc. in the sample solution. The term "to denature a nucleic acid or a nucleic acid analog" means to dissociate the base pairs. For example, it means to dissociate the base pairs formed by the mutually complementary base sequences in a molecular beacon probe to open the intramolecular structure to be in a single stranded structure, or to separate a double stranded nucleic acid molecule into single stranded nucleic acid molecules. If the fluorescent probe is an oligonucleotide including a nucleic acid analog such as PNA, it may be possible in some cases to form an associated body composed of this fluorescent probe and the target particle without performing a special denaturation treatment, even if the target particle is a double stranded nucleic acid molecule.

The denaturation treatment can be exemplified by denaturation by means of a high temperature treatment (heat denaturation), denaturation by means of a low salt concentration treatment, and the like. Of these, it is preferable to perform heat denaturation because the influence on a fluorescent substance is relatively low, and the manipulation is simple. Concretely, heat denaturation is capable of denaturing a nucleic acid or the like in this sample solution by treating this sample solution with high temperature. Generally, the denaturation can be done by keeping the temperature at 90°C for DNA and 70°C for RNA for about several seconds to two minutes, although the temperature for the denaturation is multifarious depending on the base length of the target particle etc., and it is not to be limited to this temperature as long as the denaturation is possible. On the other hand, the denaturation by means of a low salt concentration treatment can be performed by adjustment by means of dilution with, for example, purified water or the like, so that the salt concentration of this sample solution can be sufficiently low.

After the denaturation as required, the target particle and the fluorescent probe in the sample solution are associated with each other.

If the heat denaturation is performed, the target particle and the fluorescent probe in this sample solution can be suitably associated with each other by lowering the temperature of this sample solution to a temperature at which the both parties can specifically hybridize with each other, after the high temperature treatment. Moreover, if the denaturation by means of a low salt concentration treatment is performed, the target particle and the fluorescent probe in this sample solution can be suitably associated with each other by raising the salt concentration of this sample solution to a concentration at which the both parties can specifically hybridize with each other, by adding a salt solution or the like.

The temperature at which two single stranded nucleic acid molecules can specifically hybridize with each other can be obtained from the melting curve of the associated body composed of the both parties. The melting curve can be obtained by, for example, changing the temperature of a solution containing only the both parties from a high temperature to a low temperature, and measuring the absorbance or the fluorescence intensity of this solution. From the obtained melting curve, a temperature within a range from the temperature at which the two denatured single stranded nucleic acid molecules start to form an associated body to the temperature at which almost all the molecules have formed associated bodies, can be set as the temperature at which the both parties can specifically hybridize with each other. Instead of the temperature, the concentration at which two single stranded nucleic acid molecules can specifically hybridize with each other can be obtained by determining the melting curve by changing the salt concentration in the solution from a low concentration to a high concentration in the same manner.

The temperature at which the two single stranded nucleic acid molecules can specifically hybridize with each other can be generally substituted with a Tm value (melting temperature). For example, the Tm value of the region which is hybridizable with the target particle (the temperature at which 50% of double stranded DNA is dissociated into single stranded DNA) can be calculated from the base sequence information of the fluorescent probe by using a usual primer/probe design software etc.

Moreover, it is preferable to relatively slowly lower the temperature of the sample solution for forming the associated body so as to suppress non-specific hybridization. For example, after denaturing the nucleic acid molecules by setting the temperature of the sample solution at 70°C or higher, the liquid temperature of this sample solution can be lowered at a cooling rate of 0.05°C/sec. or quicker.

In addition, so as to suppress non-specific hybridization, it is also preferable to previously add a surfactant, formamide, dimethyl sulfoxide, urea, or the like, in the sample solution. A single type of, or a combination of two or more types of, these compounds may be added. By adding these compounds, non-specific hybridization can be made less likely to occur under a relatively low temperature environment.

Thereafter, as the step (b), the number of molecules of the target particles bound to the fluorescent probes existing in the prepared sample solution is counted by the scanning molecule counting method. Concretely, the sample solution upon the completion of the binding of the fluorescent probe to the target particles is set in the photoanalysis device for the scanning molecule counting method, and light emitted from the fluorescent probe in a state of being bound to the target particle is detected and analyzed by the above-mentioned technique, by which the number of molecules of the target particles bound to the fluorescent probes is counted. The counted number of molecules of the target particles bound to the fluorescent probes is the number of the target particles contained in the measurement sample.

Examples

Next is a more detailed description of embodiments of the present invention with reference to examples and the like. However, the embodiments of the present invention are not to be limited to the following examples.

[Reference Example 1]

Autofluorescent substances existing in duodenal juice collected from three examinees were measured with a detection wavelength by the scanning molecule counting method.

Of the three duodenal juice specimens used herein, one showed a pink color, another one showed a relatively strong yellow color, and the remaining one showed a very weak yellow color. Considering the difference in colors, these duodenal juice specimens were suggested to be largely different in types and amounts of auto fluorescent substances contained therein.

10 μL of each duodenal juice specimen was diluted 10-fold with Tris buffer (10 mM Tris-HCl, 1 mM EDTA, 400 mM NaCl, and pH 8.0), and this diluted solution was used as a sample solution. Each sample solution was measured with excitation light from 488 nm to 670 nm by the scanning molecule counting method. Moreover, Tris buffer, used as a reference, was also measured in the same manner by the scanning molecule counting method.

Concretely, in the measurement, a single molecule fluorescence measuring apparatus MF-20 (Olympus Corporation), equipped with the optical system of a confocal fluorescence microscope and a photon counting system was used as the photoanalysis device, to obtain the chronological photon count data of the above-mentioned respective sample solutions. At this time, laser light of 488 nm, 543 nm, 633 nm, or 670 nm was used for excitation light and irradiated at 300 μW with a rotation speed of 6,000 rpm. Regarding the signal obtained from the avalanche photodiode, BIN TIME was set to 10 μsec and the measuring time was set to 20 seconds.

The chronological data obtained by the measurement were smoothed by the Savinzky-Golay algorithm, and then peaks were detected by differentiation. Among the regions regarded as peaks, regions which were able to be approximated to the Gauss function were extracted as signals.

The measurement results are shown in Table 1. In Table 1, values shown in the column denoted by "Buffer" show the results of the measurement of Tris buffer. In Table 1, values shown in the column denoted by "Pancrease" show the results of the measurement of the 10-fold dilution of the duodenal juice specimen. In these results, in both the Tris buffer and the dilution of the duodenal juice specimen, the numbers of peaks in the cases of excitation light wavelengths of 633 nm and 670 nm were remarkably smaller than the cases of excitation light wavelengths of 488 nm and 543 nm. These results suggested that most of the autofluorescent substances contained in the duodenal juice specimen were substances emitting fluorescence having wavelengths shorter than 600 nm.

[Table 1]

| Excitation light wavelength | Buffer | | Pancrease | |
|---|---|---|---|---|
| | Number of peaks | SD | Number of peaks | SD |
| 488 nm | 80 | 11 | 46505 | 44861 |
| 543 nm | 9 | 4 | 38854 | 51849 |
| 633 nm | 1 | 1 | 1831 | 1184 |
| 670 nm | 0 | 0 | 1393 | 2321 |

[Example 1]

Samples made by adding single stranded nucleic acid molecules as the target particles to the three duodenal juice specimens used in Reference Example 1 were adopted as the biosample containing pancreatic juice to be subjected to the method for detecting a target particle of the present invention.

A single stranded nucleic acid molecule consisting of a base sequence represented by the SEQ NO: 1 was used as the target particle. Hereinunder, in this Example, this nucleic acid is referred to as the target nucleic acid. Moreover, a molecular beacon probe 1 prepared by adding TAMRA (registered trademark) to the 5'-end and BHQ-2 to the 3'-end of an oligonucleotide consisting of the base sequence represented by the SEQ NO: 2, or a molecular beacon probe 2 prepared by adding ATTO (registered trademark) 647N to the 5'-end and BHQ-2 to the 3'-end of an oligonucleotide consisting of the base sequence represented by the SEQ NO: 2, was used as the luminescent probe to be bound to this target nucleic acid. These oligonucleotides were synthesized by requesting to Sigma-Genosys Company. The base sequences of the target nucleic acid molecule and the molecular beacon probes are shown in Table 2. In Table 2, the underlined bases in the molecular beacon probes show regions which are hybridized with each other when forming the intramolecular structure.

[Table 2]

| | Base sequences | |
|---|---|---|
| Target nucleic acid | 5'-TGACTGAATATAAACTTGTGGTAGTTGGAGCTGTTGG CGTAGGCA-3' | 1 |
| Molecular beacon probe 1 | TAMRA-5'-CCTACGCCAACAGCTCCAACTACGTAGG-3' -BHQ2 | 2 |
| Molecular beacon probe 2 | ATTO647N-5'-CCTACGCCAACAGCTCCAACTACGTAGG -3'-BHQ3 | 2 |

Sample solutions were prepared by adding the target nucleic acid to a 10-fold dilution containing 10 μL of the respective duodenal juice specimen with Tris buffer (10 mM Tris-HCl, 1 mM EDTA, 400 mM NaCl, and pH 8.0) so that the final concentration would be 100 pM, 10 pM, or 0 pM, and furthermore, adding either the molecular beacon probe 1 or the molecular beacon probe 2 so that the final concentration would be 100 pM. Moreover, as reference, also prepared was a sample solution by adding the target nucleic acid to Tris buffer so that the final concentration would be 100 pM, 10 pM, or 0 pM, and furthermore, adding either the molecular beacon probe 1 or the molecular beacon probe 2 so that the final concentration would be 100 pM.

The respective sample solutions were heated at 95°C for 10 minutes, 80°C for 10 minutes, 70°C for 10 minutes, 60°C for 10 minutes, 50°C for 10 minutes, 40°C for 10 minutes, 30°C for 10 minutes, and 20°C for 10 minutes to effect an annealing treatment. By so doing, an associated body consisting of the target nucleic acid molecule A and the nucleic acid probe A was formed.

The number of molecules of the associated bodies in each sample solution after the cooling treatment was counted by the scanning molecule counting method. Concretely, the measurement was carried out in the same manner as that of Reference Example 1, except for that the 633 nm or 670 nm laser light was used for the excitation light, and that the measuring time was set to 5 seconds. The measurement results are shown in Table 3. In Table 3, values shown in the column denoted by "Buffer" show the results of the measurement of the sample solution containing no duodenal juice specimen. In Table 3, values shown in the column denoted by "Pancrease" show the results of the measurement of the sample solution containing the duodenal juice specimen. In these results, when measured with excitation light wavelengths of 543 nm, although the same amounts of the target nucleic acid were contained, greater signals were generated from the sample solution containing the duodenal juice specimen (specimen containing pancreatic juice) as compared to those from the sample solution containing no duodenal juice specimen (reference), and almost the same numbers of peaks were detected in all the sample solutions whose concentrations of the target nucleic acid were respectively 100 pM, 10 pM, and 0 pM. On the other hand, when measured with excitation light wavelengths of 633 nm, almost the same numbers of peaks were detected from the sample solution containing the duodenal juice specimen (specimen containing pancreatic juice) and from the sample solution containing no duodenal juice specimen (reference). From these results, it is apparent that many non-specific peaks were generated probably due to the influence of autofluorescent substances derived from the duodenal juice specimen, when measured with excitation light wavelengths of 543 nm; whereas, it is possible, by measuring with excitation light wavelengths of 633 nm, to suppress the generation of such non-specific peaks and to more accurately detect the target particles.

[Table 3]

| Excitation light wavelength | 543 nm | | | | 633 nm | | | |
|---|---|---|---|---|---|---|---|---|
| | Buffer | | Pancrease | | Buffer | | Pancrease | |
| | Number of peaks | SD | Number of peaks | SD | Number of peaks | SD | Number of peaks | SD |
| 100 pM | 2136 | 104 | 7461 | 225 | 4388 | 62 | 4648 | 117 |
| 10 pM | 202 | 16 | 6697 | 318 | 347 | 29 | 386 | 22 |
| 0 pM | 111 | 13 | 7220 | 802 | 260 | 26 | 360 | 237 |

INDUSTRIAL APPLICABILITY

According to the method for detecting a target particle of the present invention, pancreatic juice-derived target particles which exist only at a very low concentration in a sample solution mixed with biosample-derived autofluorescent substances can be highly precisely detected by the scanning molecule counting method. For this reason, the method for detecting a target particle of the present invention is particularly applicable to the fields such analyses/tests, and the like, in which a pancreatic juice-derived component should be the object of analysis.

Brief Description of the Reference Symbols

1: Photoanalysis device (confocal microscope)
2: Light source
3: Single mode optical fiber
4: Collimating lens
5: Dichroic mirror
6, 7, and 11: Reflective mirror
8: Object lens
9: Micro plate
10: Well (sample solution container)
12: Condenser lens
13: Pinhole
14: Barrier filter
15: Multi-mode optical fiber
16 : Photodetector
17: Mirror deflector
17a: Stage position changing apparatus

18: Computer

**Claims**

1. A method for detecting a target particle in a biosample containing pancreatic juice, wherein the method comprises:

(a) a probe-binding step for preparing a sample solution which contains a biosample containing pancreatic juice and a fluorescent probe capable of binding to a target particle, and binding the fluorescent probe to the target particle contained in the biosample, in the sample solution; and
(b) a calculation step for calculating the number of molecules of the target particles bound to the fluorescent probes existing in the sample solution prepared in the step (a);

a light emission property of light emitted from the fluorescent probe is different between a state where the fluorescent probe is bound to the target particle and a state where the fluorescent probe is present alone; and the wavelength of emitted light in a state where the fluorescent probe is bound to the target particle is 600 nm or longer; and
the calculation of the number of molecules of the target particles bound to the fluorescent probes in the step (b) being carried out by:

a moving step for, with use of an optical system of a confocal microscope or a multiphoton microscope, moving a position of a light detection region of the optical system in the sample solution;
a detection step for, while moving the position of the light detection region of the optical system in the sample solution, detecting a light signal emitted from the fluorescent probe in the state of being bound to the target particle in the light detection region, thereby individually detecting the target particles bound to the fluorescent probes; and
a counting step for counting the number of the individually detected target particles bound to the fluorescent probes to thereby count the number of molecules of the target particles detected during the moving of the position of the light detection region.

**2.** The method for detecting a target particle according to claim 1, wherein
the position of the light detection region is moved at a predetermined speed, in the moving step for moving the position of the light detection region.

**3.** A method for detecting a target particle according to either one of claim 1 and claim 2, wherein the position of the light detection region is moved at a speed higher than the speed of diffusional movement of the target particles bound to the fluorescent probes, in the moving step for moving the position of the light detection region.

**4.** The method for detecting a target particle according to any one of claim 1 to claim 3, wherein it is detected that one target particle bound to the fluorescent probe has entered the light detection region, based on the shape of the chronologically detected light signal, in the detection step for detecting light signals from the respective target particles bound to the fluorescent probes from the detected light, and individually detecting the target particles bound to the fluorescent probes.

**5.** The method for detecting a target particle according to any one of claim 1 to claim 4, wherein the wavelength of fluorescence emitted from the fluorescent probe in the state of being bound to the target particle is from 630 mn to 1300 nm.

**6.** The method for detecting a target particle according to any one of claim 1 to claim 5, wherein:

the fluorescent probe has an energy donor site and an energy acceptor site, which produce a fluorescence energy transfer phenomenon when these sites are close to each other, and the distance between the energy donor site and the energy acceptor site is different between a state where the fluorescent probe is bound to the target particle and a state where the fluorescent probe is not bound to the target particle; and
a light emission property of light emitted from the fluorescent probe is different between a state where the fluorescent probe is bound to the target particle and a state where the fluorescent probe is present alone.

**7.** The method for detecting a target particle according to any one of claim 1 to claim 6, wherein:

the target particle is a nucleic acid; and
the fluorescent probe is a single stranded nucleic acid molecule which is specifically hybridizable with the target particle, and which is bound with at least either one of a fluorescent substance serving as an energy donor and a substance serving as an energy acceptor in a fluorescence energy transfer phenomenon.

FIG. 1A

FIG. 1B

## FIG. 1C

## FIG. 2A

## FIG. 2B

## FIG. 3A

## FIG. 3B

## FIG. 4A

## FIG. 4B

## FIG. 5

```
        ╭─────────────────────────────╮
        │   Scanning molecule counting │
        │  method data analysis procedure │
        ╰─────────────────────────────╯
                      │
S100 ──┐   ┌─────────────────────────┐
        │   │  Acquisition of chronological │
        └──→│      light signal data,      │
            │   scanning of sample solution, │
            │        photon counting        │
            └─────────────────────────┘
                      │
S110 ──┐   ┌─────────────────────────┐
        └──→│   Smoothing processing of    │
            │  chronological light signal data │
            └─────────────────────────┘
                      │
S120 ──┐   ┌─────────────────────────┐
        └──→│ Time differentiation processing of chronological │
            │      light signal data after smoothing      │
            └─────────────────────────┘
                      │
S130 ──┐   ┌─────────────────────────┐
        └──→│ Detection of regions where peaks are present │
            │     Detection of regions where peaks are     │
            │        present based on data derivatives      │
            └─────────────────────────┘
                      │
S140 ──┐   ┌─────────────────────────┐
        └──→│  Fitting of bell-shaped function Fitting of  │
            │ bell-shaped function to smoothened chronological │
            │   light signal data in regions where peaks are  │
            │            present → Calculation of            │
            │            bell-function parameters            │
            └─────────────────────────┘
                      │
S150 ──┐   ┌─────────────────────────┐
        └──→│  Detection of presence of single particle │
            │         Threshold-based judgment of        │
            │ bell-shaped function parameters Removal of noise │
            └─────────────────────────┘
                      │
S160 ──┐   ┌─────────────────────────┐     ┌──────────────┐
        └──→│   Number of particles counted   │     │ To detection of │
            │       Output of bell-shaped      │     │   next peak    │
            │    function coefficient, etc.    │     └──────────────┘
            └─────────────────────────┘
                      │
S170 ──┐          ◇─────────────────◇   No
        └──────────< Processing            >──────────┘
                   < of all data completed >
                   <          ?           >
                    ◇─────────────────◇
                      │ Yes
S180 ──┐   ┌─────────────────────────┐
        └──→│  Calculation of concentration │
            │         Other analyses        │
            └─────────────────────────┘
```

## FIG. 6A

## FIG. 6B

*FIG. 7*

## INTERNATIONAL SEARCH REPORT

| International application No. |
| --- |
| PCT/JP2012/071331 |

A. CLASSIFICATION OF SUBJECT MATTER
*G01N21/64*(2006.01)i

According to International Patent Classification (IPC) or to both national classification and IPC

B. FIELDS SEARCHED

Minimum documentation searched (classification system followed by classification symbols)
G01N21/62-21/83, G01N15/00-15/14, G02B21/00

Documentation searched other than minimum documentation to the extent that such documents are included in the fields searched

| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| X<br>A | JP 2011-508219 A (Singulex, Inc.),<br>10 March 2011 (10.03.2011),<br>claim 18; paragraphs [0003], [0011], [0017] to<br>[0029], [0045], [0060], [0126], [0140]<br>& US 2009/0159812 A1    & GB 2456063 A<br>& EP 2232271 A         & WO 2009/117033 A2<br>& AU 2008352940 A     & CA 2709217 A<br>& CN 101946180 A | 1,2,5<br>3,4,6,7 |
| P,A | JP 2012-154885 A (Olympus Corp.),<br>16 August 2012 (16.08.2012),<br>claim 1<br>(Family: none) | 1-7 |

| ☐ | Further documents are listed in the continuation of Box C. | ☐ | See patent family annex. |
| --- | --- | --- | --- |

\*   Special categories of cited documents:
"A"   document defining the general state of the art which is not considered to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search<br>    10 September, 2012 (10.09.12) | Date of mailing of the international search report<br>    18 September, 2012 (18.09.12) |
| --- | --- |
| Name and mailing address of the ISA/<br>    Japanese Patent Office | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011187601 A
- JP 2005098876 A
- JP 2008292371 A
- JP 4023523 B
- WO 2008080417 A
- JP 2007020565 A

- JP 2008116440 A
- JP H04337446 B
- JP 2001198079 A
- US 20050171434 A
- JP 2010044714 A


**Non-patent literature cited in the description**

- **MASATAKA KINJO.** *Protein, Nucleic acid, and Enzyme,* 1999, vol. 44 (9), 1431-1438
- **MEYER-ALMS.** Fluorescence Correlation Spectroscopy. Springer, 2000, 204-224

- **NORIKO KATO et al.** *Gene & Medicine,* 2002, vol. 6 (2), 271-277

[Sequence listing]

SEQUENCE LISTING

<110> OLYMPUS CORPORATION, Ltd.

<120> Method for detecting a target particle in biological sample containing pancreatic juice

<130> PC-14884

<160> 2

<170> PatentIn version 3.1

<210> 1

<211> 45

<212> DNA

<213> Artificial Sequence

<220>

<223> Description of Artificial Sequence: Target oligonucleotide

<400> 1

tgactgaata taaacttgtg gtagttggag ctgttggcgt aggca          45

<210> 2

<211> 28

<212> DNA

<213> Artificial Sequence

<220>

<223> Description of Artificial Sequence: Molecular beacon probe

<400> 2

cctacgccaa cagctccaac tacgtagg          28